# DEMANDE DE BREVET EUROPEEN

(11) **EP 4 721 781 A1**
(43) Date de publication de la demande: **08.04.2026**
(21) Numéro de dépôt: 25205575.1
(22) Date de dépôt: 30.09.2025
(51) Int. Cl.: A61M 5/14

(54) **ENSEMBLE DE RÉAMÉNAGEMENT POUR PIED À PERFUSION**

(30) Priorité: 02.10.2024 BE 202400064
(71) Demandeur: Errachidi, R'Kia, 1652 Alsemberg (BE)
(72) Inventeur: Errachidi, R'Kia, 1652 Alsemberg (BE); Labbé, Jean-Pol, 1731 Zellik (BE)
(74) Mandataire: Ipsilon Belgium

(57) **Abrégé**

Ensemble de réaménagement pour pied à perfusion, en particulier pour transformer un pied à perfusion non rotatif en un pied à perfusion rotatif, le pied à perfusion comprenant un poteau monté de manière amovible sur un support, procédé de réaménagement d'un pied à perfusion non rotatif et pied à perfusion rotatif obtenu par ledit procédé de réaménagement.

## Description

### Domaine technique de l'invention

La présente invention se rapporte à un ensemble de réaménagement pour pied à perfusion, en particulier pour réaménager un pied à perfusion non rotatif et le transformer en un pied à perfusion rotatif.

### Etat de la technique

Un pied à perfusion, appelé également un pied à sérum, un porte-sérum ou un porte-perfusion, est une aide médicale utilisée pour suspendre des bouteilles, flacons, poches ou des sachets de perfusion ou de transfusion. Ces poches de perfusion sont remplies d'un médicament ou d'une solution nutritive sous forme liquide administrés à un patient par un cathéter permettant une injection continue par voie intraveineuse ou artérielle. Un tel pied à perfusion peut être utilisé en milieu hospitalier ou paramédical, ou pour continuer un traitement à domicile. Un tel pied à perfusion comprend généralement un poteau avec à son sommet une pluralité de tiges transversales, généralement pourvues d'un crochet auquel ces bouteilles de perfusion peuvent être accrochées ou attachées. Le poteau est fixé sur un support, souvent sur roulettes, permettant au patient de déambuler ou de se déplacer relativement facilement avec le pied à perfusion. Le poteau peut être réglable en hauteur.

Cependant, un problème bien connu des professionnels de la santé, en particulier par les infirmiers, par les brancardiers, par les kinésithérapeutes, et par les patients, se servant de ces pieds à perfusion, est le fait que les nombreuses tubulures reliant ces poches de perfusion au cathéter du patient s'emmêlent, s'enchevêtrent ou s'enroulent autour du poteau relativement facilement quand le patient se déplace régulièrement. Il est parfois relativement difficile de démêler ces tubulures sans débrancher ces tubulures chez le patient.

La présente invention vise donc à répondre au moins partiellement à un ou plusieurs inconvénients mentionnés ci-dessus. En particulier, l'objectif de l'invention est de fournir un ensemble de réaménagement pour pied à perfusion, en particulier pour réaménager un pied à perfusion non rotatif permettant de le transformer en un pied à perfusion rotatif.

### Résumé de l' invention

A cet effet, un premier aspect de la présente invention vise un ensemble de réaménagement pour pied à perfusion, en particulier pour réaménager un pied à perfusion non rotatif permettant de le transformer en un pied à perfusion rotatif, l'ensemble de réaménagement étant caractérisé par les éléments cités dans la revendication 1. En particulier, l'ensemble de réaménagement est destiné à réaménager un pied à perfusion non-rotatif comprenant un poteau monté de manière amovible sur un support. Le poteau est généralement monté sur le support à l'aide d'une vis et d'un écrou, ou de toute autre façon manière amovible. Le poteau est monté sur le support de façon non-rotatif. Autrement dit, le poteau ne peut pas tourner autour de son axe longitudinal une fois montée sur le support. L'ensemble de réaménagement est configuré à transformer un tel pied à perfusion non rotatif en un pied à perfusion rotatif, dans lequel le poteau pourra tourner autour de son axe longitudinal par rapport au support. Cette rotation du poteau autour de son axe est indépendante d'un mouvement rotatif potentiel du support grâce à des roulettes optionnelles. Ledit ensemble de réaménagement comprend un manchon comprenant une extrémité ouverte supérieure, une extrémité ouverte inférieure et un rebord intérieur faisant saillie vers l'intérieur. Comme défini dans un dictionnaire, un manchon est une pièce sensiblement cylindrique, tel un tube, ayant une cavité intérieure reliant les extrémités ouvertes supérieure et inférieure. Ledit manchon est configuré pour être monté sur le support. Ledit ensemble de réaménagement comprend en outre un dispositif de fixation creux configuré à être inséré au moins partiellement dans l'extrémité inférieure du manchon pour fixer le manchon sur le support, et un roulement à billes configuré à être disposé sur le rebord intérieur du manchon. Ensuite, l'ensemble de réaménagement comprend une tige configurée à être reçue dans le manchon et un moyen de séparation transversale séparant la tige en une première portion et une deuxième portion, la première portion de la tige étant configurée à être fixée au poteau, et le moyen de séparation étant configuré à se poser sur le roulement à billes. Finalement, ledit ensemble de réaménagement comprend un moyen de fixation configuré à fixer la tige au manchon. De cette façon, ledit moyen de fixation peut assurer le maintien de la tige sur le support en passant par le manchon. Ce moyen de fixation peut par exemple comprendre une paire d'un écrou et d'un contre-écrou, ou tout autre moyen de fixation connu par l'homme du métier. Grâce à cet ensemble de réaménagement, un pied à perfusion non-rotatif existant peut être transformé relativement aisément en un pied à perfusion rotatif permettant au poteau du pied à perfusion de tourner autour de son axe longitudinal, ce qui empêche au moins partiellement les tubulures attachées au pied à perfusion de s'emmêler, et/ou de démêler plus facilement ces tubulures sans devoir les détacher du patient. De plus, l'ensemble de réaménagement peut être monté et démonté relativement facilement, et permet également le remplacement relativement aisé d'une pièce défectueuse ou usée.

De manière avantageuse, ledit rebord intérieur peut être au moins partiellement incliné vers l'extrémité inférieure. Le rebord intérieur ou la saillie peut par exemple avoir la forme d'un tronc de cône inversé incluant une bride supérieure ou vers l'extérieur. De cette manière, le roulement à billes peut se poser sur ledit rebord intérieur, en particulier sur la bride, pendant que la partie intérieure du roulement à billes évite tout contact avec la partie inclinée du rebord. Ainsi, le roulement à billes peut fonctionner sensiblement sans frottement avec le manchon, ce qui permet d'éviter l'usure du roulement à billes. De préférence, le roulement à billes est seulement posé sur le rebord intérieur sans fixation au manchon, ce qui facilite l'échange et/ou le remplacement du roulement à billes si nécessaire.

Il est préférable que le dispositif de fixation creux soit une vis creuse avec filetage extérieur. La vis creuse permet le passage de la tige dans ce dispositif de fixation et le filetage extérieur peut s'engager dans un filetage intérieur correspondant dans l'extrémité inférieure du manchon pour fixer le manchon sur le support. Ce type de fixation permet un montage et/ou démontage relativement aisé et rapide. Alternativement, le dispositif de fixation peut être un raccord serré, même si la fabrication d'un tel dispositif peut être plus difficile vu la précision requise pour obtenir une fixation relativement fiable. De plus, une fixation par serrage ne permet pas un démontage aisé si nécessaire.

La tige peut par exemple comprendre un filetage extérieur et le moyen de séparation peut par exemple être un écrou vissé sur la tige. Si la tige comprend un filetage extérieur, la tige peut être montée relativement facilement au poteau du pied à perfusion à transformer. De nouveau, une fixation par vissage facilite le montage et/ou le démontage de l'ensemble de réaménagement. Si la tige comprend un filetage extérieur, un simple écrou vissé sur la tige peut faire

De manière avantageuse, le moyen de fixation peut être configuré à être disposé sur une extrémité de la deuxième portion de la tige à une distance du dispositif de fixation creux. Cette distance permet au dispositif de fixation creux de pouvoir tourner tandis que le moyen de fixation ne tourne pas quand le poteau du pied à perfusion tourne autour de son axe longitudinal.

Ledit manchon peut comprendre une portion inférieure ayant un diamètre extérieur réduit par rapport à un diamètre extérieur d'une portion supérieure du manchon. La différence de diamètre extérieur entre la portion supérieure et inférieure du manchon peut créer une surface d'appui permettant au manchon de se poser sur un support d'un pied à perfusion. Alternativement, le diamètre extérieur peut être le même sur toute la longueur du manchon et le manchon peut être joint au support d'une autre façon, en fonction de la forme du support du pied à perfusion à transformer.

Ledit manchon peut être en métal, en particulier en aluminium, et le dispositif de fixation creux peut par exemple être fait d'un alliage métallique, par exemple en laiton. Ces matériaux peuvent fournir un ensemble de réaménagement solide en restant efficace en termes de coûts de production. La combinaison de deux matériaux différents pour le manchon et le dispositif de fixation creux permet de réduire l'usure en cas de frottement. L'utilisation d'autres matériaux est possible également.

Un deuxième aspect de l'invention vise un procédé de réaménagement d'un pied à perfusion, en particulier pour un pied à perfusion non rotatif, caractérisé par les éléments cités dans les revendications 8 à 11. Ce procédé peut fournir un ou plusieurs avantages cités ci-dessus.

Un troisième aspect de l'invention vise un pied à perfusion rotatif caractérisé par les éléments cités dans la revendication 12. Ce pied à perfusion rotatif peut fournir un ou plusieurs avantages cités ci-dessus.

### Brève description des dessins

Un mode de réalisation préféré de l'invention sera décrit en référence aux dessins annexes dans lesquels
- la Figure 1 représente une vue schématique en perspective d'un mode de réalisation préféré d'un pied à perfusion ;
- la Figure 2 représente une vue schématique d'un mode de réalisation préféré d'un ensemble de réaménagement pour un pied à perfusion de la Figure 1 selon un aspect de l'invention ;
- la Figure 3a représente une vue schématique en coupe transversale du manchon de l'ensemble de réaménagement de la Figure 2 ;
- la Figure 3b représente une vue schématique en coupe transversale du dispositif de fixation creux de l'ensemble de réaménagement de la Figure 2 ;
- les Figures 4a, 4b et 4c représentent une vue schématique d'une tige avec un moyen de séparation, un roulement à billes, et un moyen de fixation de l'ensemble de réaménagement de la Figure 2 ;
- la Figure 5 représente une vue schématique de l' ensemble de réaménagement de la Figure 2 sur un pied à perfusion de la Figure 1 réaménagé.

### Description détaillée de l'invention

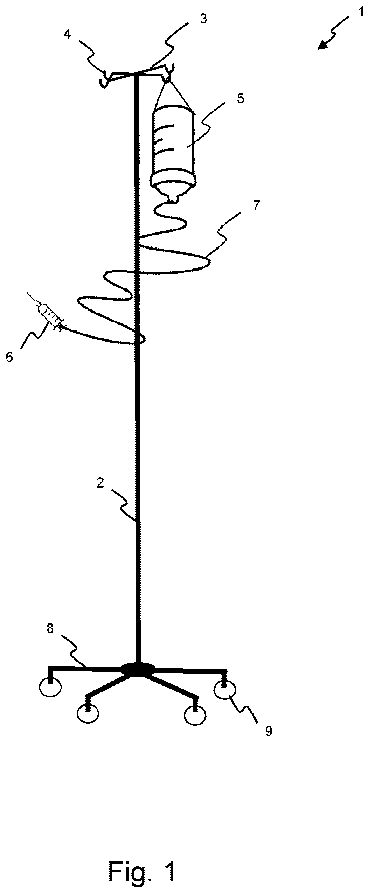

La Figure 1 représente une vue schématique en perspective d'un mode de réalisation préféré d'un pied à perfusion 1. Un pied à perfusion comprend généralement un poteau 2 avec à son sommet une pluralité de tiges transversales 3, généralement pourvues d'un crochet 4 auquel une ou plusieurs bouteilles de perfusion 5 peuvent être accrochées ou attachées. Entre cette poche de perfusion 5 et un cathéter 6 qui permet d'injecter la perfusion chez le patient, des tubulures 7 s'étendent, qui peuvent être relativement longues et qui peuvent s'enrouler autour du poteau 2, en particulier quand le pied à perfusion 1 est déplacé par rapport au patient ou quand un patient déambule avec le pied à perfusion 1. Le poteau 2 est fixé sur un support 8, qui inclut souvent des roulettes 9, permettant au patient de déambuler ou de se déplacer relativement facilement en poussant ou en tirant le pied à perfusion 1. Le poteau peut être réglable en hauteur. Généralement, le poteau 2 est monté sur le support 8 de manière amovible, par exemple via une vis en bas du poteau 2 s'étendant à travers le support et étant fixée au support 8 par un écrou ou par tout autre moyen de fixation amovible connu par l'homme du métier. Le poteau 2 est généralement monté de façon non-rotative, c'est-à-dire que l'axe longitudinal du poteau 2 ne peut servir d'axe de rotation du poteau 2 par rapport au support 8. Autrement dit, le poteau 2 ne peut tourner sur son support 8. Si le support 8 inclut des roulettes 9, le pied à perfusion peut uniquement faire une rotation sur lui-même grâce aux roulettes en gardant la position du poteau 2 fixe par rapport au support 8, ce qui peut provoquer le problème de l'enroulement des tubulures 7 autour du poteau 2. Afin de tenter de remédier à ce problème bien connu en milieu hospitalier, la présente invention vise à fournir un ensemble de réaménagement pour pied à perfusion, par exemple pour un pied à perfusion 1 comme illustré dans la Figure 1, en particulier pour transformer un pied à perfusion non rotatif en un pied à perfusion rotatif, ce qui évite aux hôpitaux le rachat de nouveaux pieds à perfusion.

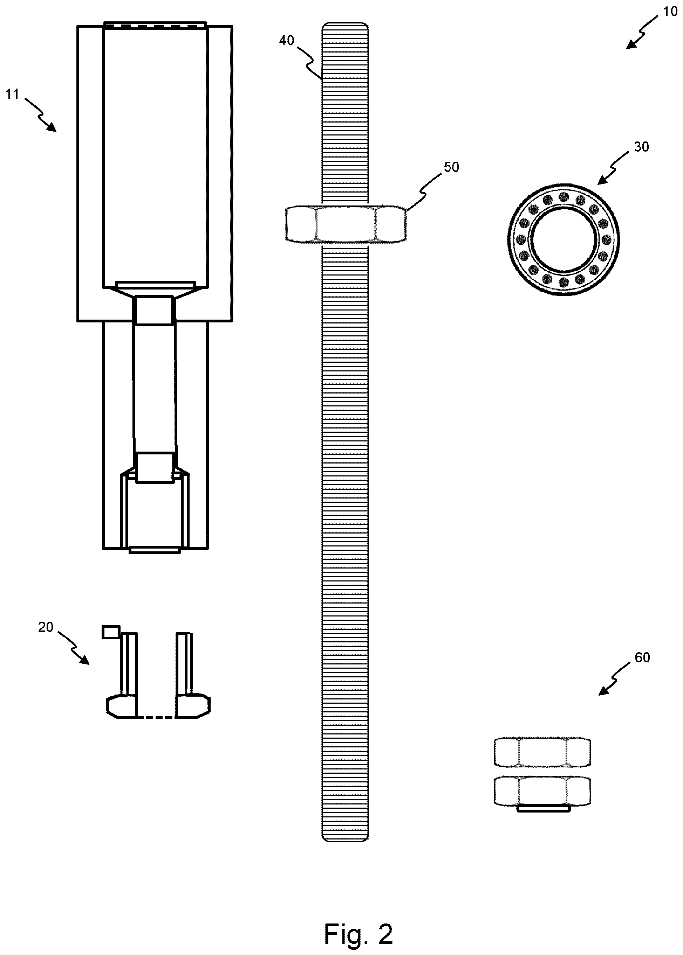

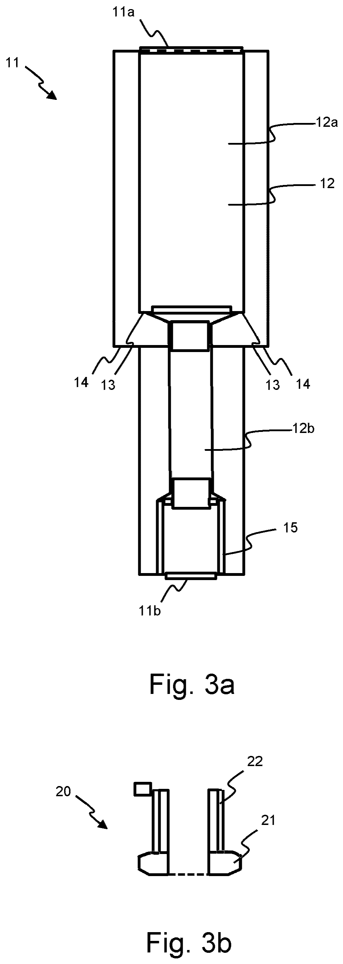

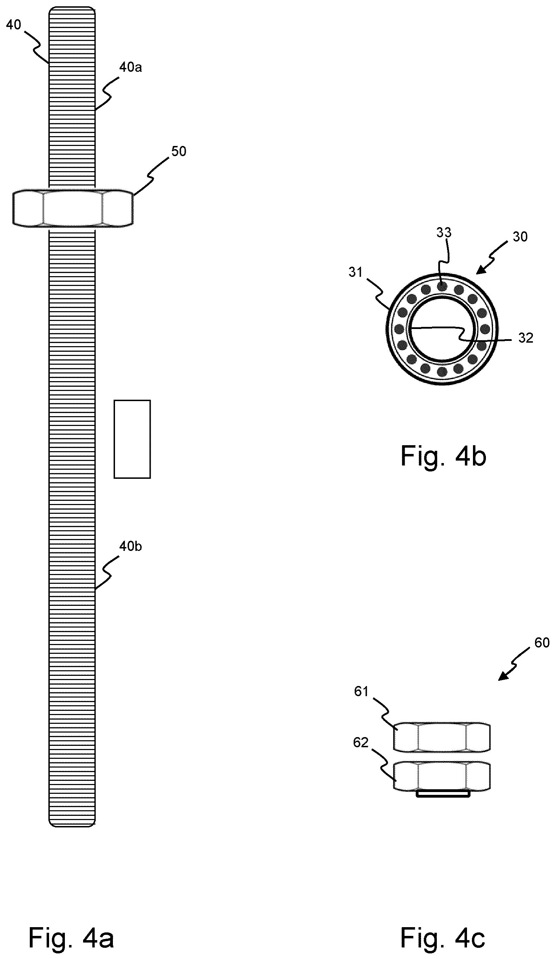

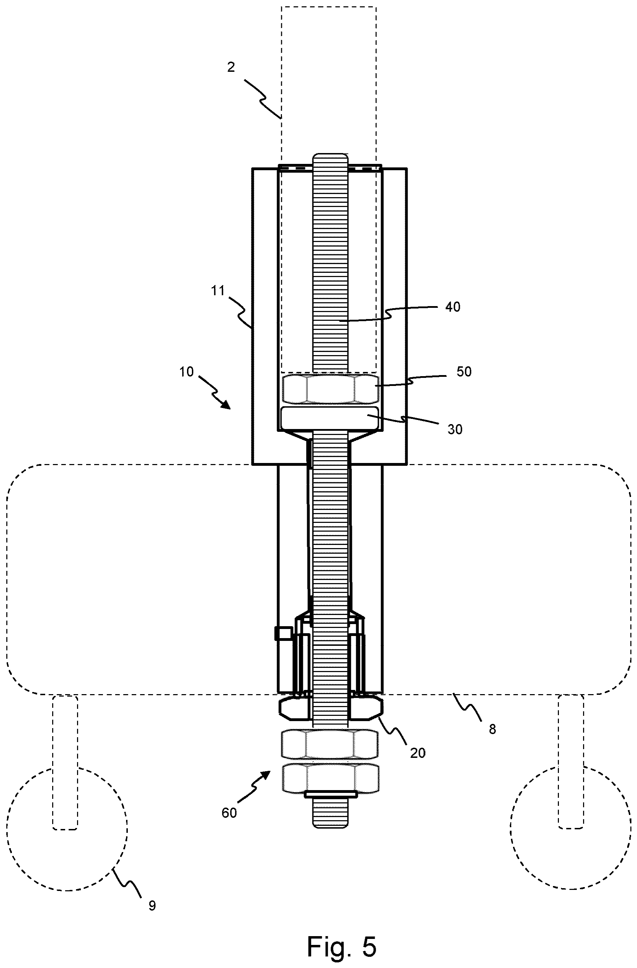

La Figure 2 représente une vue schématique d'un mode de réalisation préféré d'un ensemble de réaménagement pour un pied à perfusion de la Figure 1 selon un aspect de l'invention avant montage sur un pied à perfusion à réaménager. L'ensemble de réaménagement 10 comprend un manchon 11, un dispositif de fixation creux 20, un roulement à billes 30, une tige 40 avec un moyen de séparation 50, et un moyen de fixation 60. Les pièces seront décrites en plus de détail en référence aux Figures 3a, 3b, 4a, 4b et 4c et le procédé de réaménagement d'un pied à perfusion non rotatif en un pied à perfusion rotatif sera décrit en référence à la Figure 5.

La Figure 3a représente une vue schématique en coupe transversale du manchon 11 de la Figure 2. Le manchon 11 comprend une extrémité ouverte supérieure 11a et une extrémité ouverte inférieure 11b. Le manchon 11 est configuré pour être monté sur le support 8 d'un pied à perfusion 1, tel que représenté dans la Figure 1 ou 5, après démontage du poteau 2 du support 8. Le manchon 11 comprend une cavité centrale 12 s'étendant entre ladite extrémité ouverte supérieure 11a et ladite extrémité ouverte inférieure 11b, de préférence de forme sensiblement cylindrique. Le manchon 11 comprend un rebord intérieur 13 faisant saillie vers l'intérieur. Ledit rebord intérieur 13 peut être au moins partiellement incliné vers l'extrémité inférieure 11b. De préférence, le rebord intérieur 13 comprend une première partie sensiblement transversale à la paroi intérieure du manchon et une deuxième partie inclinée de façon à ce que la première partie puisse servir d'appui pour une partie extérieure du roulement à billes 30 et la deuxième partie inclinée puisse être espacée du roulement à billes 30, tel que montré dans la Figure 5. Ledit rebord intérieur 13 ou saillie peut avoir une forme d'un cône tronqué inversé. Le rebord intérieur 13 peut se situer plus ou moins vers le milieu du manchon et permet de définir une partie supérieure 12a de la cavité centrale 12 du manchon 11 et une partie inférieure 12b. La partie supérieure 12a peut être configurée à recevoir une portion du poteau 2 du pied à perfusion 1, comme illustré dans la Figure 5. Cette portion supérieure 12a peut avoir un diamètre interne plus large qu'une partie inférieure 12b de la cavité centrale 12 ayant un diamètre plus réduit. Le rebord intérieur 13 peut former la transition entre ces diamètres différents. Un diamètre interne de la partie supérieure 12a de la cavité centrale peut par exemple être compris entre 2 cm et 3 cm, étant par exemple plus ou moins 2.5 cm. Le diamètre interne peut être choisi en fonction du diamètre du poteau 2 du pied à perfusion 1 à réaménager. Le diamètre interne de la partie inférieure 12b peut par exemple être compris entre plus ou moins 0.5 cm et 2 cm, par exemple plus ou moins 1 cm, et peut être choisi en fonction du diamètre de la tige 40, telle que représentée dans la Figure 4a. L'extrémité ouverte inférieure 11b est configurée pour recevoir le dispositif de fixation creux, tel que montré dans la Figure 3b. A cette fin, ladite ouverture inférieure 11b peut comprendre un filet interne 15. Le diamètre externe du manchon 11 peut être le même le long du manchon ou peut varier, comme montré dans la Figure 3, où un diamètre extérieur supérieur est plus large qu'un diamètre extérieur inférieur, le diamètre extérieur supérieur étant par exemple compris entre 3 cm et 4 cm, étant par exemple plus ou moins 3.5 cm tandis que le diamètre extérieur inférieur peut être compris entre 2 cm et 3 cm, étant par exemple plus ou moins 2.5 cm ou étant sensiblement le même que le diamètre extérieur supérieur. Si le diamètre extérieur supérieur est plus grand que le diamètre extérieur inférieur, le manchon 11 peut également, mais ne doit pas nécessairement, inclure un rebord extérieur 14 formant un moyen d'appui du manchon 11 quand le manchon 11 est inséré dans le support 8, comme illustré dans la Figure 5.

La Figure 3b représente une vue schématique en coupe transversale du dispositif de fixation creux de l'ensemble de réaménagement de la Figure 2. Ce dispositif de fixation creux est configuré à être inséré au moins partiellement dans l'extrémité inférieure 11b du manchon 11 pour fixer le manchon 11 sur le support 8, comme illustré dans la Figure 5. Le dispositif 20 est creux afin de permettre à la tige 40 de s'étendre à travers ledit dispositif 20. Ledit dispositif de fixation creux 20 peut par exemple être une vis creuse avec filetage extérieur. Les dimensions du dispositif de fixation 20 dépendent de préférence des dimensions du manchon 11. En particulier, le dispositif 20 peut comprendre une tête 21 dont le diamètre est élargi, étant par exemple plus large que le diamètre inférieur du manchon 11 pour fixer le manchon 11 sur le support 8. Ledit diamètre de la tête 21 peut par exemple être compris entre 2 cm et 2.5 cm, étant par exemple 2.2 cm. Le dispositif 20 de fixation creux peut être fixé à l'extrémité inférieure 11b du manchon 11 par vissage, ou alternativement, par serrage. Le dispositif 20 peut par exemple inclure un filetage extérieur 22 correspondant au filetage 15 du manchon 11. Le manchon 11 et le dispositif de fixation 20 peuvent être fabriqués en métal, par exemple en aluminium ou en laiton, ou en d'autres matériaux connus de l'homme du métier. De préférence, le manchon 11 est en aluminium, et le dispositif de fixation creux 20 est en laiton.

Les Figures 4a, 4b et 4c représentent une vue schématique d'une tige avec un moyen de séparation, un roulement à billes, et un moyen de fixation de l'ensemble de réaménagement de la Figure 2. La tige 40 est configurée à être reçue dans le manchon 11. Le moyen de séparation transversale 50 peut par exemple être un écrou qui est monté ou vissé sur la tige 40 si la tige est une tige filetée à l'extérieur. Le moyen de séparation 50 est configuré à séparer la tige 40 en une première portion 40a et une deuxième portion 40b, la première portion 40a de la tige étant configurée à être fixée au poteau 2 du pied à perfusion à réaménager. La deuxième portion 40b de la tige 40 est configurée à s'étendre à travers la partie inférieure 12b du manchon 11. Le moyen de séparation 50 est configuré à se poser sur le roulement à billes 30. Le roulement de billes 30 comprend généralement un bord extérieur 31 et un bord intérieur 32 entre lesquels se trouvent une pluralité de billes 33. Le bord extérieur 31 est configuré à être posé sur le rebord intérieur 13. Le bord intérieur 32 est configuré à être en contact avec la tige 40 qui est fixée au poteau 2 du pied à perfusion 1, ce qui permet au poteau 2 de tourner autour de son axe longitudinal. L'ensemble de réaménagement 10 comprend finalement un moyen de fixation configuré à fixer la tige 40 au manchon 11. Ce moyen de fixation 60 peut par exemple comprendre un écrou 61 et un contre-écrou 62 pour éviter que l'écrou 61 se dévisse. Les dimensions de ce moyen de fixation 60 peuvent être choisies en fonction des dimensions de la tige 40. Le moyen de fixation 60 est de préférence configuré à être disposé sur une extrémité de la deuxième portion 40b de la tige 40 à une distance du dispositif de fixation creux 20 pour ne pas bloquer la rotation de la tige 40 entrainant le poteau 2 et le dispositif de fixation creux 20.

La Figure 5 représente une vue schématique de l'ensemble de réaménagement de la Figure 2 sur un pied à perfusion de la Figure 1 réaménagé, résultant en un pied à perfusion rotatif. Les éléments faisant partie du pied à perfusion 1 à réaménager sont indiqués en pointillés, comme le poteau 2, le support 8 et les roulettes 9 optionnelles. Le procédé de réaménagement comprend d'abord une étape de démontage partiel du pied à perfusion 1, en particulier de démontage du poteau 2 dudit support 8, qui est généralement vissé sur le support 8. Puis, le manchon 11 de l'ensemble de réaménagement 10 est inséré dans le support 8, en particulier dans le trou central du support dans lequel se trouvait le poteau 2 avant démontage. Le manchon 11 peut être inséré par exemple jusqu'à ce qu'un rebord extérieur 14 se pose sur le support 8. Ensuite, le dispositif de fixation creux 20 est au moins partiellement inséré, de préférence vissé, dans l'extrémité inférieure ouverte 11b du manchon 11 pour fixer le manchon 11 sur le support 8. Puis, la première portion 40a de la tige 40 peut être fixée à une première extrémité du poteau 2, par exemple par vissage. Le roulement à billes 30 peut être disposé sur le rebord intérieur 13 du manchon 11. Alternativement, le roulement à billes 30 peut être monté sur la deuxième portion 40b de la tige 40, contre le moyen de séparation 50. Ensuite, la tige 40, qui est fixée au poteau 2, est inséré dans l'extrémité ouverte supérieure 11a du manchon 11 jusqu'à ce que le moyen de séparation transversale 50 soit posé sur le roulement à billes 30. Finalement, la tige 40 est fixée au manchon 11 par un moyen de fixation 60, de préférence en vissant un écrou 61 et un contre-écrou 62 sur une extrémité de la tige filetée 40. Ce moyen de fixation 60 est de préférence disposé sur une extrémité de la deuxième portion 40b de la tige 40 à une distance du dispositif de fixation creux 20 pour éviter tout engagement entre ce moyen de fixation 60 et le dispositif de fixation 20.

Même si la présente invention a été illustrée en référence à des modes de réalisation spécifiques, l'homme du métier comprendra que l'invention n'est pas limitée aux détails des modes de réalisations illustratifs, et que la présente invention peut être réalisée avec de nombreuses modifications sans s'écarter de la portée de l'invention. Les modes de réalisation doivent être considérés comme illustratifs et non de façon restrictive, la portée de l'invention étant définie par les revendications qui suivent plutôt que par la description qui précède. Toute modification qui entre dans la signification ou l'équivalence des revendications est destinée à être comprise. Autrement dit, il est envisagé de couvrir toutes les modifications, variations ou équivalences qui tombent sous la portée des principes de base sous-jacents et dont les caractéristiques essentielles sont revendiquées dans cette demande de brevet. Le lecteur de cette demande de brevet comprendra que les mots "comprenant" ou "comprend" n'excluent pas d'autre élément ou étape, et que les mots "un" ou "une" n'excluent pas une pluralité. Les signes de références dans les revendications ne peuvent être considérés comme limitant la revendication concernée. Les termes "premier", "deuxième", "troisième", "a", "b", "c", etcetera sont introduits pour distinguer différents éléments ou étapes et ne décrivent pas nécessairement un ordre séquentiel ou chronologique. De même, les termes "supérieure", "inférieure", "dessus", "dessous", etcetera sont introduits à des fins descriptives et pas nécessairement pour désigner des positions relatives. On comprendra que ces termes sont interchangeables sous des conditions appropriées et que des modes de réalisations de l'invention sont capables d'être opérables selon la présente invention dans d'autres séquences ou dans des orientations qui diffèrent de celles décrites ou illustrées ci-dessus.

## Revendications

1. Ensemble de réaménagement pour pied à perfusion, en particulier pour transformer un pied à perfusion non rotatif en un pied à perfusion rotatif, le pied à perfusion comprenant un poteau monté de manière amovible sur un support, l'ensemble de réaménagement comprenant
a. un manchon comprenant une extrémité ouverte supérieure, une extrémité ouverte inférieure et un rebord intérieur faisant saillie vers l'intérieur, le manchon étant configuré pour être monté sur le support ;
b. un dispositif de fixation creux configuré à être inséré au moins partiellement dans l'extrémité inférieure du manchon pour fixer le manchon au support ;
c. un roulement à billes configuré à être disposé sur le rebord intérieur du manchon ;
d. une tige configurée à être reçue dans le manchon et un moyen de séparation transversale séparant la tige en une première portion et une deuxième portion, la première portion de la tige étant configurée à être fixée au poteau, et le moyen de séparation étant configuré à se poser sur le roulement à billes ;
e. un moyen de fixation configuré à fixer la tige au manchon.

2. Ensemble selon la revendication 1, dans lequel ledit rebord intérieur est au moins partiellement incliné vers l'extrémité inférieure.

3. Ensemble selon l'une quelconque des revendications précédentes, dans lequel le dispositif de fixation creux est une vis creuse avec filetage extérieur.

4. Ensemble selon l'une quelconque des revendications précédentes, dans lequel la tige comprend un filetage extérieur et le moyen de séparation est un écrou vissé sur la tige.

5. Ensemble selon l'une quelconque des revendications précédentes, dans lequel le moyen de fixation est configuré à être disposé sur une extrémité de la deuxième portion de la tige à une distance du dispositif de fixation creux.

6. Ensemble selon l'une quelconque des revendications précédentes, dans lequel ledit manchon comprend une portion inférieure ayant un diamètre extérieur réduit par rapport à un diamètre extérieur d'une portion supérieure du manchon.

7. Ensemble selon l'une quelconque des revendications précédentes, dans lequel ledit manchon est en métal, en particulier en aluminium, et le dispositif de fixation creux est en laiton.

8. Procédé de réaménagement d'un pied à perfusion non rotatif comprenant les étapes de
∘ fournir un pied à perfusion non rotatif comprenant un poteau monté de manière amovible sur un support;
∘ fournir un ensemble de réaménagement selon l'une quelconque des revendications 1 à 7 ;
∘ démonter le poteau dudit support ;
∘ insérer le manchon dans le support ;
∘ insérer le dispositif de fixation creux au moins partiellement dans l'extrémité inférieure du manchon pour fixer le manchon sur le support ;
∘ disposer le roulement à billes sur le rebord intérieur du manchon ;
∘ fixer la première portion de la tige à une première extrémité du poteau ; ∘ insérer la tige fixée au poteau dans l' extrémité ouverte supérieure du manchon jusqu'à ce que le moyen de séparation transversale soit posé sur le roulement à billes ;
∘ fixer la tige au manchon par un moyen de fixation.

9. Procédé de réaménagement selon la revendication 8, dans lequel le dispositif de fixation creux est vissé au moins partiellement dans l'extrémité inférieure du manchon.

10. Procédé de réaménagement selon l'une quelconque des revendications précédentes 8 - 9, dans lequel le moyen de fixation est disposé sur une extrémité de la deuxième portion de la tige à une distance du dispositif de fixation creux.

11. Procédé de réaménagement selon l'une quelconque des revendications précédentes 8 - 10, dans lequel la première portion de la tige est vissée dans une première extrémité du poteau.

12. Pied à perfusion rotatif obtenu par le procédé de réaménagement selon l'une quelconque des revendications précédentes 8 à 11.
